# EUROPEAN PATENT SPECIFICATION

(11) **EP 3 613 415 B1**
(45) Date of publication and mention of the grant of the patent: **09.11.2022**
(21) Application number: 18788015.8
(22) Date of filing: 26.03.2018
(51) Int. Cl.: A61K 8/73, A61K 8/81, A61K 8/90, A61K 9/14, A61K 31/506, A61K 31/517, A61K 31/44, A61K 31/496, C10M 169/04, C10N 20/06, C10N 20/00, A61K 8/02, A61Q 19/00

(54) **METHOD FOR PREPARING ACTIVE MATERIAL NANOPARTICLES USING LIPID AS LUBRICANT FOR MILLING**
VERFAHREN ZUR HERSTELLUNG VON AKTIVMATERIAL-NANOPARTIKELN UNTER VERWENDUNG VON LIPID ALS SCHMIERMITTEL ZUM FRÄSEN
PROCÉDÉ DE PRÉPARATION DE NANOPARTICULES DE MATÉRIAU ACTIF À L'AIDE DE LIPIDE EN TANT QUE LUBRIFIANT EN VUE DE BROYAGE

(30) Priority: 21.04.2017 KR 20170051600
(43) Date of publication of application: 26.02.2020
(73) Proprietor: Bio-Synectics Inc., Seoul 08501 (KR)
(72) Inventor: KIM, Kab Sig, Seoul 03477 (KR); LEE, Eun Yong, Seoul 08748 (KR); KANG, Si On, Gwangmyeong-si Gyeonggi-do 14230 (KR); CHOI, Jae Woo, Seocho-gu Seoul 06679 (KR); KIM, Jeong Kyu, Osan-si Gyeonggi-do 18108 (KR); PARK, Joo Won, Seoul 08717 (KR); LEE, Won Suk, Suwon-si Gyeonggi-do 16670 (KR); JIN, Yong Suk, Suwon-si Gyeonggi-do 16666 (KR)
(74) Representative: Schrell, Andreas
(86) International application number: PCT/KR2018/003560
(87) International publication number: WO 2018/194283

(56) References cited:
- EP-A1- 2 013 138
- EP-A2- 2 481 396
- WO-A1-2010/121326
- KR-A- 20050 054 819
- KR-A- 20090 027 734
- KR-A- 20110 060 285
- KR-A- 20110 106 247
- KR-A- 20130 031 479
- US-A1- 2007 071 826

## Description

### TECHNICAL FIELD

The present invention relates to a method for preparing nanoparticle of active ingredient using lipid as lubricant in milling process, and more specifically, it relates to a method for preparing active ingredient into nanoparticle, which can be properly used in drugs, cosmetics, functional foods, etc., by pulverizing mixture comprising the active ingredient, a lipid as a lubricant, and a biocompatible polymer having a glass transition temperature of 80°C or higher through milling process, and then removing the lipid used as a lubricant therefrom by using supercritical fluid.

### BACKGROUND ART

A demand for a technique of effective and rapid preparation of very fine particles in regular size has been constantly required in various industrial fields. Such fine particles in regular size have many advantages, particularly among which good flowability and little deviation in particle interaction are very advantageous in industrial application. For example, in drug industry, the particle size of a therapeutic agent greatly affects the dissolution rate, bioavailability, formulation and the like, and as the deviation in the interaction between the particles of a therapeutic agent becomes smaller, the overall stability of the therapeutic agent becomes better.

In medicinal products, if the particle of a therapeutic agent is made into nanoscale size, the following advantages are obtained. First of all, for drugs having a small enteral absorption rate in oral administration, more absorption can be achieved and thus the bioavailability of the therapeutic agent can be increased, as compared with those of a bigger size. Furthermore, the dosage form of drugs can be various. For instance, a drug that has been administered only via oral route may be administered by inhalation. In a controlled-release drug formulation, the release rate of a therapeutic agent is a very important factor. When the particle size of the therapeutic agent is formed in nanoscale, the particle size becomes relatively more uniform and thus the release rate can become more expectable, thereby being possible to provide more effective therapeutic agent.

Since uniform nanoparticles have various advantages as described above, many attempts have been made to prepare an active ingredient as a nanoparticle. Conventionally, mechanical techniques such as crushing, grinding, milling and the like have been employed to make large particles relatively smaller. Recently in the pharmaceutical industry, a method using an air-jet mill is generally used to pulverize a large amount of therapeutic agent to the size range being suitable for medicinal use. However, according to US Patent No. 5,534,270 and Lachman, et al. [The Theory and Practice of Industrial Pharmacy, Chapter 2, "Milling", p.45, (1986)], such conventional mechanical processes have been generally recognized as having a limitation of possible minimum particle size of about tens of micrometers.

Keiji Yamamoto et al. asserted that nanoparticles of drug may be prepared by pulverizing the drug along with cyclodextrin using a rod mill [Chem, Pharm, Bull. 55(3)359-363 (2007)]. They asserted that the amount of cyclodextrin used in this method is about two times of the active ingredient in molar ratio, i.e. about four times in weight ratio, and that humidity for hydrating all used cyclodextrin is needed and it is disadvantageous if the humidity is too high or too low.

US Patent No. 5,145,684 discloses a method for preparing particles of a poorly water-soluble drug in a size of hundreds of nanometers by wet-milling the poorly water-soluble drug in the presence of a surfactant. This technique should be applied after preparing the drug in a particle size of 100 micrometers or less by using a conventional pulverizing process. In this method, the time for preparing particles within the target size range depends on the mechanical device used therefor. When a ball mill is used, 5 days or longer is required. However, when a high shear media mill is used, the particles can be prepared within 1 day. However, since the nanoparticle obtained in this method is in a suspension phase, in order to make it in powder type, a process of spray dry or freeze dry should be conducted. During these processes, however, coagulation of particles occurs and when the obtained powder is re-dispersed in liquid, it is difficult to obtain a dispersion of particles in nanometer scale substantially. In order to solve such a problem, US Patent No. 5,302,401 discloses an anti-coagulation agent employed during lyophilization. Additionally, US Patent No. 6,592,903 B2 discloses use of a stabilizer, a surfactant and an anti-coagulation agent during a spray dry process. Furthermore, US Patent Application Publication No. 2003/0185869 A1 discloses an application of a wet milling technique using lysozyme as an interface stabilizer to some poorly soluble drugs. However, in this case, since the interface stabilizer is a protein, there are many restrictions in drying and accordingly only the preparation in liquid phase is disclosed.

US Patent Application Publication No. 2002/0168402 A1 discloses a method for preparing nanoparticle using piston gap homogenization. However, in order to use piston gap homogenization, a pretreatment process using jet mill or hammer mill for pulverizing particle into uniform size is required. In addition, because this process is not available for highly viscose solution, it should be performed in a state where the concentration of active gradient is low.

As another conventional method, there is a recrystallization technique which provides fine particles of active ingredient by changing the environment of a solution containing the active ingredient dissolved therein to cause precipitation or crystallization of solute. The recrystallization technique can be practiced in two different ways: the one being comprised of dissolving a therapeutic agent in a suitable solvent and lowering the temperature, thereby changing the solubility of the therapeutic agent to precipitate particles; and the other being comprised of adding antisolvent to a solution containing the therapeutic agent dissolved therein, thereby decreasing the dissolving ability of the solvent to precipitate particles. However, most of such recrystallization techniques usually require use of organic solvent harmful to human, and flocculation or coagulation of the particles in wet condition occurs during a drying process after filtration of the precipitated particles. As a result, the final particles may not be uniform in size.

US Patent Application Publication No. 2003/0104068 A1 discloses a method for preparing fine particles by dissolving a polymer in an organic solvent, dissolving or dispersing a protein drug therein, rapidly cooling the solution to ultra-low temperature for solidification, and lyophilizing the product to provide fine powder. In this case, however, the protein drug may be denatured by the contact with an organic solvent, and the process needs the rapid cooling and lyophilizing processes and thus it is not economical.

In addition, there are techniques of reducing particle size by using emulsification. Such emulsifying methods are commonly used in cosmetic field, and provide fine particles by melting poorly water-soluble substances by heat or dissolving them in an organic solvent, and adding the melted or dissolved substances to an aqueous solution containing a surfactant dissolved therein, with stirring at high speed or with sonication to disperse the added substances. However, in this case, a step for removing water is required to provide fine particles in powder form, and many restrictions are generated during the water removal step. Furthermore, when an organic solvent is used to dissolve the poorly water-soluble substance, there always is a concern to the residual organic solvent harmful to human.

US Patent Application Publication No. 2004/0067251 A1 discloses a method for preparing fine particles by dissolving an active ingredient in an organic solvent and spraying the resulting solution into an aqueous solution containing a surfactant dissolved therein. This method uses an organic solvent, and since the prepared particles exist in an aqueous phase, a drying process is required for removing water used as solvent, to provide the particles in powder form. During the drying process, however, the coagulation of the particles occurs and thus it is hard to re-disperse them in nanoscale size.

Recently, many attempts have been made to use supercritical fluid in preparing amorphous or nanoscale particles. Supercritical fluid is a fluid existing in liquid form at a temperature higher than its critical temperature and under pressure higher than its critical pressure. Commonly used supercritical fluid is carbon dioxide. As one of the methods using supercritical fluid in preparing nanoparticles, rapid expansion of a supercritical solution ("RESS," hereinafter) has been known [Tom et al. Biotechnol. Prog. 7(5):403-411. (1991); US Patent No. 6,316,030 B1; and US Patent No. 6,352,737 B1]. According to this method, a target solute is firstly dissolved in supercritical fluid, and then the supercritical solution is rapidly sprayed into a relatively low-pressure condition via nozzle. Then, the density of the supercritical fluid rapidly falls down. As a result, the ability of the supercritical fluid to solubilize the solute is also rapidly reduced, and the solute is formed into very fine particles or crystalline.

Other techniques using supercritical fluid include a gas-antisolvent recrystallization ("GAS," hereinafter) [Debenedetti et al. J. Control. Release 24:27-44. (1993); WO 00/37169]. This method comprises dissolving a therapeutic agent in a conventional organic solvent to prepare a solution and spraying the solution through a nozzle into a supercritical fluid serving as an antisolvent. Then, rapid volume expansion occurs due to the contact between the solution and the supercritical fluid. As a result, the density and dissolving capacity of the solvent decrease, and thereby extreme supersaturation is caused and nuclei or particles of the solute are formed. Further methods for preparing active ingredient nanoparticles are disclosed in US 2007/071826 A1, EP 2 013 138 A1 and EP 2 481 396 A2.

As stated above, various techniques or preparing active ingredient into nanoparticles have been developed, but there have been limitations in their application. In addition, they have many limitations in terms of economy.

### DETAILED DESCRIPTION OF THE INVENTION

### TECHNICAL PURPOSE

The present invention seeks to solve the above-mentioned problems of the prior arts. The purpose of the present invention is to provide a method capable of effectively preparing active ingredient in nanoparticle size through milling process, while overcoming the limitation in application and the low economic capacity as in the prior arts.

### TECHNICAL MEANS

Accordingly, the present invention provides a method for preparing nanoparticle of active ingredient comprising: (1) a step of providing a mixture comprising the active ingredient, a lipid as a lubricant, and a biocompatible polymer having a glass transition temperature of 80°C or higher; (2) a step of pulverizing the resulting product of said step (1) through milling process; and (3) a step of removing the lipid from the resulting product of said step (2) by using supercritical fluid, wherein the lipid is used in an amount of five parts by weight or less, based on one part by weight of the active ingredient.

In an embodiment of the present invention, the above step (1) may be a step of physically and uniformly mixing the active ingredient, a lipid as a lubricant, and a biocompatible polymer having a glass transition temperature of 80°C or higher.

In an embodiment of the present invention, the above step (1) may be a step of mixing the active ingredient and a lipid as a lubricant; and to this mixture, a biocompatible polymer having a glass transition temperature of 80°C or higher is added together with demineralized water; and then physically and uniformly mixing the resulting product.

In an embodiment of the present invention, the above step (1) may be a step of physically and uniformly mixing the active ingredient, a lipid as a lubricant, a biocompatible polymer having a glass transition temperature of 80°C or higher, and one or more additional components selected from a biocompatible polymer having a glass transition temperature of lower than 80°C, a surfactant, and an anti-coagulation agent.

In an embodiment of the present invention, the above step (1) may be a step of mixing the active ingredient and a lipid as a lubricant; and to this mixture, a biocompatible polymer having a glass transition temperature of 80°C or higher, and one or more additional components selected from a biocompatible polymer having a glass transition temperature of lower than 80°C, a surfactant, and an anti-coagulation agent are added together with demineralized water; and then physically and uniformly mixing the resulting product.

In an embodiment of the present invention, the above step (1) may be a step of physically and uniformly mixing a solidified mixture and a biocompatible polymer having a glass transition temperature of 80°C or higher, wherein the solidified mixture was prepared by pouring a solution, where the active ingredient and a lipid as a lubricant are dissolved in water miscible organic solvent (having a property of being mixed with water), into water for solidification; filtering and drying the mixture.

In an embodiment of the present invention, the above step (1) may be a step of physically and uniformly mixing a solidified mixture and a biocompatible polymer having a glass transition temperature of 80°C or higher with demineralized water, wherein the solidified mixture was prepared by pouring a solution, where the active ingredient and a lipid as a lubricant are dissolved in water miscible organic solvent, into water for solidification; filtering and drying the mixture.

In an embodiment of the present invention, the above step (1) may be a step of physically and uniformly mixing a solidified mixture, a biocompatible polymer having a glass transition temperature of 80°C or higher, and one or more additional components selected from a biocompatible polymer having a glass transition temperature of lower than 80°C, a surfactant, and an anti-coagulation agent, wherein the solidified mixture was prepared by pouring a solution, where the active ingredient and a lipid as a lubricant are dissolved in water miscible organic solvent, into water for solidification; filtering and drying the mixture.

In an embodiment of the present invention, the above step (1) may be a step of physically and uniformly mixing a solidified mixture, a biocompatible polymer having a glass transition temperature of 80°C or higher, and one or more additional components selected from a biocompatible polymer having a glass transition temperature of lower than 80°C, a surfactant, and an anti-coagulation agent together with demineralized water, wherein the solidified mixture was prepared by pouring a solution, where the active ingredient and a lipid as a lubricant are dissolved in water miscible organic solvent, into water for solidification; filtering and drying the mixture.

In an embodiment of the present invention, the above step (1) may be a step of preparing a solidified mixture by pouring a solution, where the active ingredient, the lipid as a lubricant, and the biocompatible polymer having a glass transition temperature of 80°C or higher are dissolved in water miscible organic solvent, into water for solidification; and filtering and drying the mixture.

In an embodiment of the present invention, the above step (1) may be a step of physically and uniformly mixing a solidified mixture and one or more additional components selected from a biocompatible polymer having a glass transition temperature of lower than 80°C, a surfactant, and an anti-coagulation agent, wherein the solidified mixture was prepared by pouring a solution, where the active ingredient, a lipid as a lubricant, and a biocompatible polymer having a glass transition temperature of 80°C or higher are dissolved in water miscible organic solvent, into water for solidification; and filtering and drying the mixture.

In an embodiment of the present invention, the above step (1) may be a step of physically and uniformly mixing a solidified mixture and one or more additional components selected from a biocompatible polymer having a glass transition temperature of lower than 80°C, a surfactant, and an anti-coagulation agent together with demineralized water, wherein the solidified mixture was prepared by pouring a solution, where the active ingredient, a lipid as a lubricant, and a biocompatible polymer having a glass transition temperature of 80°C or higher are dissolved in water miscible organic solvent, into water for solidification; and filtering and drying the mixture.

In the above step (1), the physical mixing may be performed by a mixer conventionally used, without special limitation, such as v-mixer, vertical mixer, ribbon mixer, planetary mixer, roll mill or the like.

In the above step (1), there is no special limitation in the temperature of mixing the components, and for example, the mixing may be performed at 40°C or lower (e.g., 10 to 40°C), and preferably at 30°C or lower (e.g., 10 to 30°C).

In an embodiment of the present invention, the milling process in the above step (2) may be performed continuously by using counter rotating rolls (for example, 2-roll mill or 3-roll mill).

In an embodiment of the present invention, the lipid removal in the above step (3) may be performed by continuously adding supercritical fluid into a reactor containing the resulting product of said step (2) and discharging it therefrom.

In an embodiment of the present invention, the lipid removal using supercritical fluid in the above step (3) may be performed under a pressure condition of 5.1 MPa (50 atmospheres) or higher and a temperature condition of 5 to 60°C.

### EFFECTS OF THE INVENTION

The method for preparing nanoparticle of active ingredient according to the present invention uses a lipid as a lubricant when milling a mixture comprising active ingredient, a biocompatible polymer, and optionally a surfactant and/or an anti-coagulation agent by using a roll mill (for example, 2-roll mill or 3-roll mill) so as to perform the milling process smoothly, thereby allowing production of nanoparticle of active ingredient using a roll mill in commercial scale. The nanoparticles of active ingredient prepared according to the present invention have very good dispersability, absorption, physiological activity, etc. and thus can be properly used in drugs, functional foods, general foods, cosmetics, etc.

### BRIEF EXPLANATION OF DRAWINGS

Figure 1 shows the particle size distributions of the nanoparticles prepared in Examples 1 to 5.
Figure 2 shows the particle size distributions of the nanoparticles prepared in Examples 6 and 7.
Figure 3 shows the particle size distribution of the nanoparticles prepared in Example 8.
Figure 4 shows the pXRD analysis result of the nanoparticles prepared in Example 10.
Figure 5 shows the pXRD analysis results of the nanoparticles prepared in Example 10 and Comparative Example in comparison.
Figure 6 shows the particle size distribution of the nanoparticles prepared in Example 11.
Figure 7 shows the particle size distributions of the nanoparticles prepared in Examples 12 and 13.
Figure 8 shows the particle size distributions of the nanoparticles prepared in Examples 15 and 16.
Figure 9 shows the particle size distributions of the nanoparticles prepared in Examples 17 to 19.

### CONCRETE CONTENTS FOR CARRYING OUT THE INVENTION

The present invention is explained in more detail below.

In the present invention, the active ingredient is a material that exhibits physiological activity in, for example, medicinal products, functional foods, cosmetics and the like, and as the active ingredient, one or more selected from physiologically active organic compounds, organometallic compounds, natural extracts, proteins, and combinations thereof can be used, but it is not limited thereto. There is no special limitation to its state at room temperature such as solid phase or liquid phase, and its electrical form such as neutral or ionic form.

According to specific embodiments of the present invention, as physiologically active material, salt, isomer, ester, ether or other derivative thereof, the active ingredient may include, for example, anticancer agents, antifungal agents, analgesics, psychiatric agents, consciousness level-altering agents such as anesthetic agents or hypnotics, nonsteroidal antiinflammatory agents, anthelminthics, antiacne agents, antianginal agents, antiarrhythmic agents, anti-asthma agents, antibacterial agents, anti-benign prostatic hypertrophy agents, anticoagulants, antidepressants, antidiabetics, antiemetics, antiepileptics, antigout agents, antihypertensive agents, antiinflammatory agents, antimalarials, antimigraine agents, antimuscarinic agents, antineoplastic agents, antiobesity agents, antiosteoporosis agents, antiparkinsonian agents, antiproliferative agents, antiprotozoal agents, antithyroid agents, antitussive agent, anti-urinary incontinence agents, antiviral agents, anxiolytic agents, appetite suppressants, beta-blockers, cardiac inotropic agents, chemotherapeutic drugs, cognition enhancers, contraceptives, corticosteroids, Cox-2 inhibitors, diuretics, erectile dysfunction improvement agents, expectorants, gastrointestinal agents, histamine receptor antagonists, immunosuppressants, keratolytics, lipid regulating agents, leukotriene inhibitors, macrolides, muscle relaxants, neuroleptics, nutritional agents, opioid analgesics, protease inhibitors, or sedatives, etc. but it is not limited thereto.

As used herein, the term "nanoparticle(s)" refers to particle(s) wherein 90% or more of the particles have a mean size of 5µm or less, preferably 2µm or less, more preferably 1µm or less (for example, 0.9µm or less, 0.8µm or less, 0.7µm or less, or 0.6µm or less), still more preferably 0.5 µm or less, still more preferably 0.4 µm or less, still more preferably 0.3 µm or less, and still more preferably 0.2 µm or less. The lower limit of the mean size of the nanoparticles may be 1 nm or greater, or 5 nm or greater, or 10 nm or greater, or 50 nm or greater, but it is not limited thereto.

In the present invention, there is no special limitation to the state of the lipid used as a lubricant, and any of liquid phase to solid phase thereof at room temperature can be used. In an embodiment, a lipid having good solubility to supercritical fluid can be used preferably, and an example of such a lipid may be selected from saturated fatty acids having 10 to 22 carbon atoms, esters of saturated fatty acid having 10 to 22 carbon atoms, saturated fatty alcohols having 10 to 22 carbon atoms, mono-, di or tri-glycerides having saturated fatty acid group having 10 to 22 carbon atoms, hydrocarbons having 14 or more (for example, 14 to 24) carbon atoms, and combinations thereof, but it is not limited thereto.

According to specific embodiments of the present invention, for example, the lipid may be selected from fatty alcohols such as myristyl alcohol, cetyl alcohol, stearyl alcohol and lauryl alcohol, fatty acids such as stearic acid, palmitic acid, myristic acid and lauric acid, and their esters with methyl, ethyl, propyl and butyl alcohol, etc., fatty acid monoglycerides such as stearyl monoglyceride, palmityl monoglyceride, myristyl monoglyceride, lauryl monoglyceride, etc., fatty acid diglycerides such as distearyl glyceride, dipalmityl glyceride, dimyristyl glyceride, dilauryl glyceride, etc., hydrocarbons such as tetradecane, pentadecane, hexadecane, heptadecane, octadecane, nonadecane, icosane, heneicosane, docosane, tricosane, tetracosane, etc., and combinations thereof, but it is not limited thereto at all. According to the present invention, any lipid can be used as long as it serves as a lubricant in the milling process and can be removed by supercritical fluid.

In the present invention, according to claim 1, based on 1 part by weight of the active ingredient, the lipid can be used in an amount of 0.1 part by weight or more, or 0.3 part by weight or more, or 0.5 part by weight or more. In addition, based on 1 part by weight of the active ingredient, the lipid can be used in an amount of 3 parts by weight or less, or 2 parts by weight or less, or 1.5 parts by weight or less. If the use amount of the lipid is too little as compared with the active ingredient, heavy load is applied in the following milling process and thus it is difficult to perform smooth milling, or the active ingredient can be changed to amorphous form or other crystal form and thus the stability of the prepared nanoparticles may deteriorate. To the contrary, if the use amount of the lipid is too much as compared with the active ingredient, the economy of the following lipid removal process may deteriorate seriously. In addition, when the lipid to be used is in a liquid phase at room temperature, the use amount thereof may be 1.5 parts by weight or less, based on 1 part by weight of the active ingredient, since if the use amount is too much, the state of the mixture becomes slurry, and thus the milling may not be performed properly.

If active ingredient is prepared as fine particles such as nanoparticles, the surface energy increases and according to elapsed time, the case of coagulation or crystal growth into larger particles happens frequently. In order to prevent such coagulation or crystal growth, the glass transition temperature of the biocompatible polymer used in the nanoparticle preparation process becomes a very important factor. If the glass transition temperature of the biocompatible polymer used in the nanoparticle preparation process is lower than 80°C, the coagulation and/or crystal growth of the prepared nanoparticles cannot be prevented sufficiently, and thus the size of the prepared particles becomes larger according to elapsed time. In order to prevent such coagulation and/or crystal growth of the prepared nanoparticles, the present invention necessarily uses a biocompatible polymer having a glass transition temperature of 80°C or higher (preferably, 90°C or higher).

For example, the biocompatible polymer having a glass transition temperature (Tg) of 80°C or higher (preferably, 90°C or higher), which can be used in the present invention, may be one or more selected from cellulose-based biocompatible polymer having a Tg of 80°C or higher such as methylcellulose (MC) (e.g., MC having a Tg of about 184 to 197°C), hydroxyethylcellulose (HEC) (e.g., HEC having a Tg of about 127°C), hydroxypropylcellulose (HPC) (e.g., HPC having a Tg of about 105°C), hydroxypropyl methylcellulose (HPMC) (e.g., HPMC having a Tg of about 180°C), hydroxypropyl methylcellulose acetate succinate (HPMC-AS) (e.g., HPMC-AS having a Tg of about 117°C), hydroxypropyl methylcellulose phthalate (HPMC-P) (e.g., HPMC-P having a Tg of about 145°C), carboxymethyl cellulose (CMC) (e.g., CMC having a Tg of about 135°C), cellulose acetate (CA) (e.g., CA having a Tg of about 180°C), dextran (e.g., dextran having a Tg of about 200°C), etc., polyvinyl pyrrolidone (PVP) having a Tg of 80°C or higher (e.g., PVP k17, k30, k90 having a Tg of about 138 to 156°C), polyvinyl alcohol having a Tg of 80°C or higher, and Eudragit-based biocompatible polymer having a Tg of 80°C or higher.

The biocompatible polymer having a Tg of 80°C or higher necessarily used in the present invention may be used alone or in combination of two or more kinds. The use amount thereof is preferably 0.01 part by weight or more, based on 1 part by weight of the active ingredient. More concretely, the biocompatible polymer having a Tg of 80°C or higher can be used in amount of 0.05 part by weight or more, 0.1 part by weight or more, 0.15 part by weight or more, 0.2 part by weight or more, 0.25 part by weight or more, or 0.3 part by weight or more, based on 1 part by weight of the active ingredient, and it can be used in amount of 5 parts by weight or less, 3 parts by weight or less, 2 parts by weight or less, 1 parts by weight or less, or 0.5 part by weight or less, based on 1 part by weight of the active ingredient.

The one or more additional components selected from a biocompatible polymer having a glass transition temperature of lower than 80°C, a surfactant and an anti-coagulation agent, which can be optionally used in the present invention, are those used in medicinal products, foods and cosmetics, and there is no special limitation to its electrical property (e.g., ionic, nonionic, etc.) and there is no special limitation to its state at room temperature (e.g., liquid phase, wax or solid phase, etc.), and it can be used alone or in combination of two or more kinds.

There is no special limitation to the additional component which can be optionally used in the present invention, and any of biocompatible polymers having a glass transition temperature of lower than 80°C, surfactants and anti-coagulation agents known as useful for nanoparticle preparation of active ingredient, or any of novel ones which can be used for nanoparticle preparation of active ingredient, can be employed in the present invention. Such an optional additional component may be, for example, a biocompatible polymer having a glass transition temperature (Tg) of lower than 80°C such as gelatin, casein, gum acacia, tragacanth, polyethylene glycols, poloxamers, eudragit^{®} having a Tg of lower than 80°C, lysozyme, albumin, etc.; a surfactant such as cetyl pyridinium chloride, phospholipids, fatty acid, benzalkonium chloride, calcium stearate, glycerin esters of fatty acid, fatty alcohol, cetomacrogol, polyoxyethylene alkyl ethers, sorbitan esters, polyoxyethylene castor oil derivatives, polyoxyethylene sorbitan fatty acid esters, dodecyl trimethyl ammonium bromide, polyoxyethylene stearate, sodium lauryl sulfate, sucrose fatty acid ester, PEG-cholesterol, PEG-vitamin E, etc.; an anti-coagulation agent such as saccharide, etc. In the present invention, the saccharide is of a concept including monosaccharide compounds, disaccharide compounds, polysaccharide compounds, sugar alcohols and the like, particularly including glucose, lactose, mannitol, sucrose, xylitol, chitosan, starch fiber and the like, and it can be used alone or in a mixture form. The optional additional component can be used alone or in combination of two or more kinds, but it is not limited to the above concrete examples at all.

In the present invention, in case of using one or more of the optional additional components, each of them may be used, for example, in amount of 0.001 part by weight or more, 0.005 part by weight or more, or 0.01 part by weight or more, based on 1 part by weight of the active ingredient, and it may be used in amount of 5 parts by weight or less, 3 parts by weight or less, 2 parts by weight or less, 1 parts by weight or less, or 0.5 part by weight or less, based on 1 part by weight of the active ingredient.

There is no special limitation to the water miscible organic solvent which can be used in the present invention, as long as it is a solvent which can dissolve the active ingredient and the lipid used as a lubricant, and can be mixed with water and can prepare a mixture wherein the active ingredient and the lipid used as a lubricant are mixed homogeneously in water.

Concretely, the water miscible organic solvent may be selected from, for example, mono or polyhydric alcohol and amine having 8 or less (e.g., 1 to 8) carbon atoms, ketone such as acetone, dimethyl sulfoxide (DMSO), dimethylformamide (DMF), tetrahydrofurane (THF), pyridine and combinations thereof, but it is not limited thereto.

In the present invention, in case of using the water miscible organic solvent, it may be used in amount of 0.1 part by weight or more, 0.5 part by weight or more, or 1 part by weight or more, based on 1 part by weight of the active ingredient. In addition, the water miscible organic solvent may be used in amount of 10 parts by weight or less, 6 parts by weight or less, or 3 parts by weight or less, based on 1 part by weight of the active ingredient. If the use amount of the water miscible organic solvent is too little as compared with the active ingredient, it may be hard to dissolve the active ingredient and the lipid as a lubricant at a low temperature (for example, 100°C or lower). To the contrary, if the use amount of the water miscible organic solvent is too much as compared with the active ingredient, a considerable amount of the active ingredient cannot be solidified during the solidification in water, and thus the production yield may become low.

In the method for preparing nanoparticle of the present invention, step (1) provides a mixture comprising the active ingredient, a lipid as a lubricant, and a biocompatible polymer having a glass transition temperature of 80°C or higher.

In an embodiment, the above step (1) may be a step of physically and uniformly mixing the active ingredient, a lipid as a lubricant, and a biocompatible polymer having a glass transition temperature of 80°C or higher, and optionally one or more additional components selected from a biocompatible polymer having a glass transition temperature of lower than 80°C, a surfactant, and an anti-coagulation agent.

In an embodiment, the above step (1) may be a step of mixing the active ingredient and a lipid as a lubricant; and to this mixture, a biocompatible polymer having a glass transition temperature of 80°C or higher is added together with demineralized water; and then physically and uniformly mixing the resulting product.

In an embodiment, the above step (1) may be a step of mixing the active ingredient and a lipid as a lubricant; and to this mixture, a biocompatible polymer having a glass transition temperature of 80°C or higher, and optionally one or more additional components selected from a biocompatible polymer having a glass transition temperature of lower than 80°C, a surfactant, and an anti-coagulation agent are added together with demineralized water; and then physically and uniformly mixing the resulting product.

In an embodiment of the present invention, the above step (1) may be a step of physically and uniformly mixing a solidified mixture and a biocompatible polymer having a glass transition temperature of 80°C or higher, wherein the solidified mixture was prepared by pouring a solution, where the active ingredient and a lipid as a lubricant are dissolved in water miscible organic solvent (having a property of being mixed with water), into water for solidification; filtering and drying the mixture.

In an embodiment of the present invention, the above step (1) may be a step of physically and uniformly mixing a solidified mixture and a biocompatible polymer having a glass transition temperature of 80°C or higher with demineralized water, wherein the solidified mixture was prepared by pouring a solution, where the active ingredient and a lipid as a lubricant are dissolved in water miscible organic solvent, into water for solidification; filtering and drying the mixture.

In an embodiment of the present invention, the above step (1) may be a step of physically and uniformly mixing a solidified mixture, a biocompatible polymer having a glass transition temperature of 80°C or higher, and optionally one or more additional components selected from a biocompatible polymer having a glass transition temperature of lower than 80°C, a surfactant, and an anti-coagulation agent, wherein the solidified mixture was prepared by pouring a solution, where the active ingredient and a lipid as a lubricant are dissolved in water miscible organic solvent, into water for solidification; filtering and drying the mixture.

In an embodiment of the present invention, the above step (1) may be a step of physically and uniformly mixing a solidified mixture, a biocompatible polymer having a glass transition temperature of 80°C or higher, and optionally one or more additional components selected from a biocompatible polymer having a glass transition temperature of lower than 80°C, a surfactant, and an anti-coagulation agent together with demineralized water, wherein the solidified mixture was prepared by pouring a solution, where the active ingredient and a lipid as a lubricant are dissolved in water miscible organic solvent, into water for solidification; filtering and drying the mixture.

In an embodiment of the present invention, the above step (1) may be a step of preparing a solidified mixture by pouring a solution, where the active ingredient, the lipid as a lubricant, and the biocompatible polymer having a glass transition temperature of 80°C or higher are dissolved in water miscible organic solvent, into water for solidification; and filtering and drying the mixture.

In an embodiment of the present invention, the above step (1) may be a step of physically and uniformly mixing a solidified mixture and optionally one or more additional components selected from a biocompatible polymer having a glass transition temperature of lower than 80°C, a surfactant, and an anti-coagulation agent, wherein the solidified mixture was prepared by pouring a solution, where the active ingredient, a lipid as a lubricant, and the biocompatible polymer having a glass transition temperature of 80°C or higher are dissolved in water miscible organic solvent, into water for solidification; and filtering and drying the mixture.

In an embodiment of the present invention, the above step (1) may be a step of physically and uniformly mixing a solidified mixture and optionally one or more additional components selected from a biocompatible polymer having a glass transition temperature of lower than 80°C, a surfactant, and an anti-coagulation agent together with demineralized water, wherein the solidified mixture was prepared by pouring a solution, where the active ingredient, a lipid as a lubricant, and a biocompatible polymer having a glass transition temperature of 80°C or higher are dissolved in water miscible organic solvent, into water for solidification; and filtering and drying the mixture.

When the crystallinity of the active ingredient should be maintained or the active ingredient is sensitive to heat, each of the components may be fed into a mixer in powder state, and mixed uniformly. If it is necessary to mix the active ingredient and the lipid more uniformly, the active ingredient and the lipid are fed into a mixer, the water miscible organic solvent is added thereto and the mixture is heated for clear dissolution, and then the resulting solution is poured into water at a temperature, which is lower than the melting point of the lipid used as a lubricant-for example, into demineralized distilled water at 40°C or lower, preferably 30°C or lower, more preferably 25°C or lower, still more preferably 20°C or lower-for solidification, and the obtained solid (including waxy form) is filtered and dried under reduced pressure to yield a mixture powder wherein the components are mixed uniformly.

The biocompatible polymer, surfactant, and/or anti-coagulation agent may be added in powder state, together with demineralized water, or in solution form, according to the cases.

In the above step (1), in case of adding the biocompatible polymer having a glass transition temperature of 80°C or higher and/or the optional additional component together with demineralized water and physically mixing them uniformly, there is no special limitation to the amount of water used, but for smoothness and economy of the process, it is preferable to use water in amount of 40%(w/w) or less (more concretely, 35%(w/w) or less, 30%(w/w) or less, 25%(w/w) or less, 20%(w/w) or less, or 15%(w/w) or less), based on the total weight of the mixture including water. In order to achieve effective pulverization in the milling process of step (2), strong shearing force should be transferred to the mixture, and the shearing force is closely associated with the water content in the mixture. If the water content is high, the shearing force becomes very low and the pulverization is not performed effectively, and the economy becomes deteriorate since it takes much time to reduce the water content to the level under which the shearing force capable of effective pulverization can be transferred.

In the method for preparing nanoparticle of the present invention, in step (2), the resulting product of the above step (1) (that is, a mixture comprising the active ingredient, a lipid as a lubricant, and a biocompatible polymer having a glass transition temperature of 80°C or higher, and, if necessary, optional additional component) is pulverized through milling process. The milling process may be performed continuously, for example, by using 2-roll mill or 3-roll mill.

Roll mill is a device wherein two or more rolls (for example, 2-roll mill or 3-roll mill, etc.) counter rotate, and apply compressing and shearing force to the fed mixture to pulverize it. In the present invention, any type of roll mill can be used, as long as it is a roll mill which can apply compressing and shearing force to the resulting product of the above step (1) to pulverize it.

During the milling process, the gap between the rolls may be 1 mm or less, for example, 500 µm or less, 200 µm or less, preferably 100 µm or less, more preferably 50 µm or less, and still more preferably 30 µm or less. If the gap between the rolls is too broad, the compressing and shearing force becomes low and it is difficult to obtain sufficient pulverizing effect, and if the gap between the rolls is too narrow, there may be a problem of lowering the feeding speed. A skilled artisan in this field of art can adjust the gap easily and suitably in production spot.

The temperature of the roll in the milling process may be adjusted suitably according to the melting point and amount of the used lubricant, i.e., the lipid, and the amount of water used in adding the biocompatible polymer, etc. In an embodiment, if the amount of lipid used is 1 part by weight or more, based on 1 part by weight of the active ingredient, it may be preferable to maintain the temperature of the roll as lower than the melting point of the used lipid. In another embodiment, if the amount of lipid used is less than 1 part by weight, based on 1 part by weight of the active ingredient, the temperature of the roll may be set to be around the melting point (melting point±5°C) of the lipid used or higher. In other case, for example, if a lipid in liquid phase at room temperature such as lauryl alcohol (melting point: 22°C) is used, it would be advantageous for the milling process to adjust the amount of lipid added (e.g., 0.1 to 1 part by weight, or 0.2 to 0.8 part by weight, or 0.3 to 0.5 part by weight, based on 1 part by weight of the active ingredient), rather than the temperature of the roll.

Even if the biocompatible polymer having a glass transition temperature of 80°C or higher and/or optional additional component are added simply or added together with demineralized water, if it is intended to perform the milling under moisture condition of 1%(w/w) or lower by promoting evaporation of water during the milling process, the milling process is performed at the melting point of the lipid used as a lubricant or lower (for example, in a temperature range of 0 to 20°C lower than the melting point of the lipid) until the active ingredient becomes nanoparticles sufficiently. At this time, if there is no lipid, too high shearing force is applied to the mixture of the active ingredient and the biocompatible polymer and the milling process is hard to perform, and thus the commercial production is impossible.

If it is intended to add the biocompatible polymer having a glass transition temperature of 80°C or higher and/or optional additional component are added together with demineralized water to the mixture of the active ingredient and the lipid, and pulverize it by using the shearing force increase according to the moisture reduction, it is advantageous for nanoparticle preparation to perform the milling process with at the roll temperature of 35°C or lower, preferably 25°C or lower, more preferably 20°C or lower, and still more preferably 17°C or lower. If the milling process is performed repeatedly, the water content in the mixture becomes lower and lower, and if the water content becomes 20%(w/w) or lower, preferably 15%(w/w) or lower, more preferably 12%(w/w) or lower, and still more preferably 10%(w/w) or lower, the shearing force in the milling process increases rapidly and the nanoparticle preparation of active ingredient becomes easy. Whereas, the glass transition temperature of the biocompatible polymer having a glass transition temperature of 80°C or higher present in the mixture increases rapidly as the water content in the mixture becomes lower, and if there is no lipid used as a lubricant, heavy load is applied to roll during the milling process and thus economical production is very hard.

In the method for preparing nanoparticle of the present invention, in step (3), the lipid used as a lubricant is removed from the resulting product of the above step (2) by using supercritical fluid. In an embodiment, the lipid removal may be performed by continuously adding supercritical fluid into a reactor containing the resulting product of the above step (2) and discharging it therefrom. This may be performed, for example, under a pressure condition of 50 atmospheres or higher and a temperature condition of 5 to 60°C.

As used herein, the term "supercritical fluid" refers to a gas or liquid, which is inert with no reactivity such as carbon dioxide or nitrogen, and can be a supercritical fluid under specific temperature and specific pressure, i.e., supercritical temperature and supercritical pressure.

In addition, as used herein, the term "critical pressure" refers to specific pressure, under a pressure of which or higher a gas of supercritical fluid can become supercritical fluid.

In an embodiment, the solid mixture obtained in the above step (2) is placed in a high pressure reactor, and while maintaining the inside of the reactor at a temperature not allowing the lipid used as a lubricant to flow down for example, 5 to 60°C, more concretely 10 to 40°C, a gas of supercritical fluid (for example, carbon dioxide) is added into the reactor to pressurize the inside of the reactor, for example, to 5.1 MPa to 40.5 MPa (50 to 400 atmospheres), preferably 6.1 MPa to 20.3 MPa (60 to 200 atmospheres) and then the supercritical fluid is continuously added into the inside of the reactor and discharged to the outside of the reactor with controlling the addition valve and the discharge valve, thereby the lipid used as a lubricant is discharged together with the supercritical fluid to the outside of the reactor and removed. At this time, of the temperature of the inside of the high pressure reactor is too high, the lipid used as a lubricant may act as a solvent to the active ingredient, and as a result, the crystal of the active ingredient prepared in nanoparticle size may grow. Thus, it is preferable to maintain the temperature of the reactor within a range capable of decreasing the liquidity of the lipid present in the solid mixture obtained in step (2) as low as possible, preferably at a temperature of the melting point of the lipid or lower, and considering the workability, it is preferable to maintain the temperature as 10 to 40°C.

In addition, the time for removing the lipid with supercritical fluid depends on the kind and amount of the used lipid, and in order to obtain the active ingredient particles with higher purity, it is preferable to minimize the amount of residual lipid by removing the lipid for sufficient time as possible. The lipid preferably used in the present invention is not harmful to human body, and thus it is not necessary to limit its residual amount to a specific range, but considering the purity of the obtained active ingredient, the residual amount is preferably less than 1 % by weight of the total weight. In case of using a lipid such as mono-, di- or tri-glyceride group compound, which can also be used as a surfactant, there may be no problem even if the residual amount is greater than 10 % by weight.

The lipid removed from the solid mixture powder as above can be collected in a separate reactor and be subsequently used in the following production process.

The present invention is explained in detail through the following Examples and Comparative Examples. However, the scope of the present invention is not limited thereto.

### Examples 1 to 5

As an active ingredient, 1 g of Nilotinib (free base) was added into a beaker together with 2 g of the lipid shown in the following Table 1, and mixed well with spatula. Then, 0.3 g of polyvinylpyrrolidone (PVP k30) and 0.05 g of sodium lauryl sulfate (SLS) were added thereto and further mixed well. To the resulting mixture, continuous milling was performed using 3-roll mill (TRX-3100S; Intec system) under the conditions shown in the following Table 1 to obtain a solid dispersion type mixture wherein Nilotinib, lipid as a lubricant, and PVP k30, and SLS were mixed uniformly.

The obtained solid dispersion type mixture was placed in a high pressure reactor, and carbon dioxide as supercritical fluid was continuously added thereto at 15 to 25°C under 7.1 MPa to 10.1 MPa (70 to 100 atmospheres) to remove the lipid used as a lubricant, and a mixture powder, wherein Nilotinib, PVP k30, and SLS were mixed, was obtained.

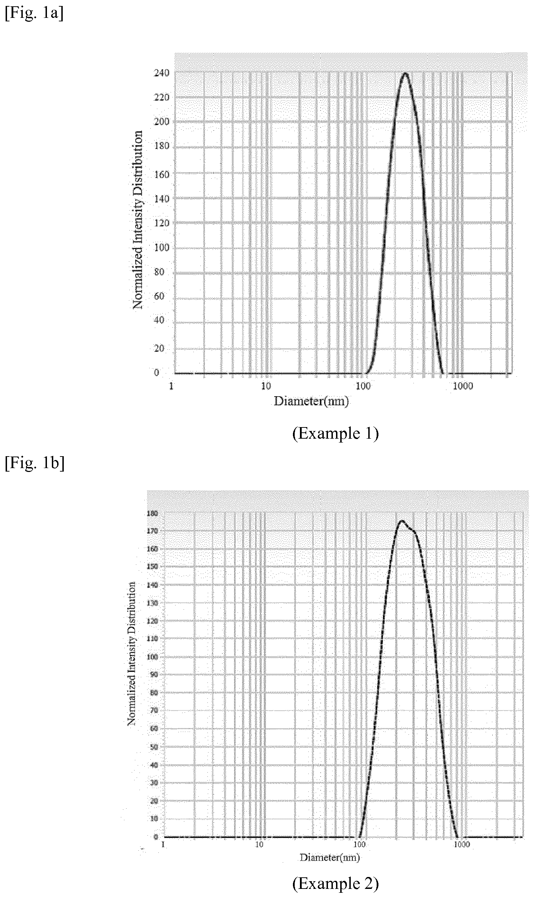

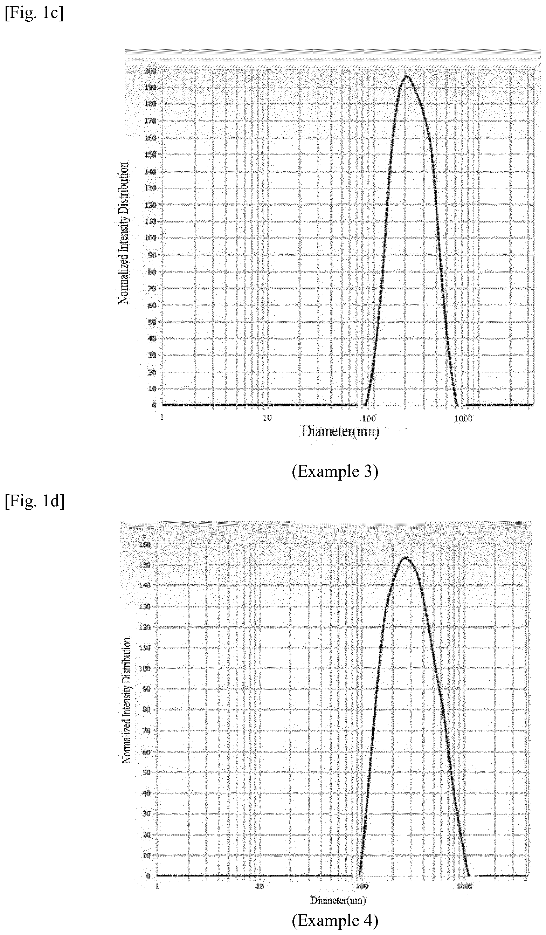

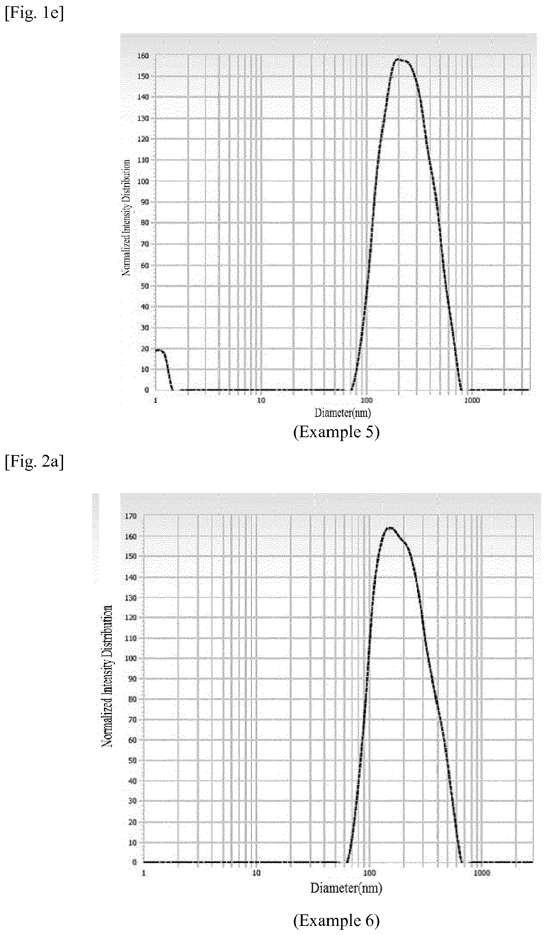

The obtained powder was dispersed in demineralized distilled water at a concentration of 1 mg/ml (based on Nilotinib), and then the particle size was measured with ELSZ-1000 (Otsuka) in DLS (Dynamic light scattering) manner. The particle size distributions of the nanoparticles prepared in Examples 1 to 5 are shown in Figure 1.

**[Table 1]**

| Example | Active ingredient | Lipid | PVP k30 | SLS | Roll mill conditions | | | Mean particle size (nm) | Particle size distribution (PDI) |
|---|---|---|---|---|---|---|---|---|---|
| | | | | | R.S (rpm) | R.T (°C) | O.N (number) | | |
| 1 | 1 g | MA 2 g | 0.3 g | 0.05 g | 60 | 27 to 30 | 70 | 238.9 | 0.165 |
| 2 | 1 g | CA 2 g | 0.3 g | 0.05 g | 60 | 35 to 38 | 70 | 227.0 | 0.164 |
| 3 | 1 g | SA 2 g | 0.3 g | 0.05 g | 60 | 47 to 49 | 70 | 204.4 | 0.183 |
| 4 | 1 g | CA 2 g | 0.3 g | 0.05 g | 60 | 27 to 30 | 20 | 255.2 | 0.222 |
| 5 | 1 g | SA 2 g | 0.3 g | 0.05 g | 60 | 27 to 30 | 28 | 210.8 | 0.227 |

| | | | | | | | | | |
|---|---|---|---|---|---|---|---|---|---|
| Active ingredient: Nilotinib MA: Myristyl alcohol (melting point: 40°C) CA: Cetyl alcohol (melting point: 49.3°C) SA: Stearyl alcohol (melting point: 59.4°C) R.S: Roller speed R.T: Roller temperature O.N: Number of roll mill operation | | | | | | | | | |

In case of Examples 1 to 3, the roller temperature (R.T) was maintained as about 10°C (±5°C) lower than the melting point of the respective lipid, the milling was performed smoothly, and heavy load was not applied to the roll during the milling. In case of Examples 4 and 5, the milling was performed under the conditions wherein the roller temperature compared with the melting point of the lipid was lower than those of Examples 1 to 3, and although some load was applied to the roll, the nanoparticles were prepared well.

### Examples 6 and 7

As an active ingredient, 1 g of Nilotinib (free base) was mixed with 0.3 g and 0.5 g, respectively, of lauryl alcohol (LA) well, and 0.3 g of PVP k30 and 0.05 g of SLS were added thereto and further mixed well. To the resulting mixture, continuous milling was performed using 3-roll mill (TRX-3100S; Intec system) under the conditions shown in the following Table 2 to obtain a solid dispersion type mixture wherein Nilotinib, lipid as a lubricant, and PVP k30, and SLS were mixed uniformly.

The obtained solid dispersion type mixture was placed in a high pressure reactor, and carbon dioxide as supercritical fluid was continuously added thereto at 15 to 25°C under 7.1 MPa to 10.1 MPa (70 to 100 atmospheres) to remove the lipid used as a lubricant, and a mixture powder, wherein Nilotinib, PVP k30, and SLS were mixed, was obtained.

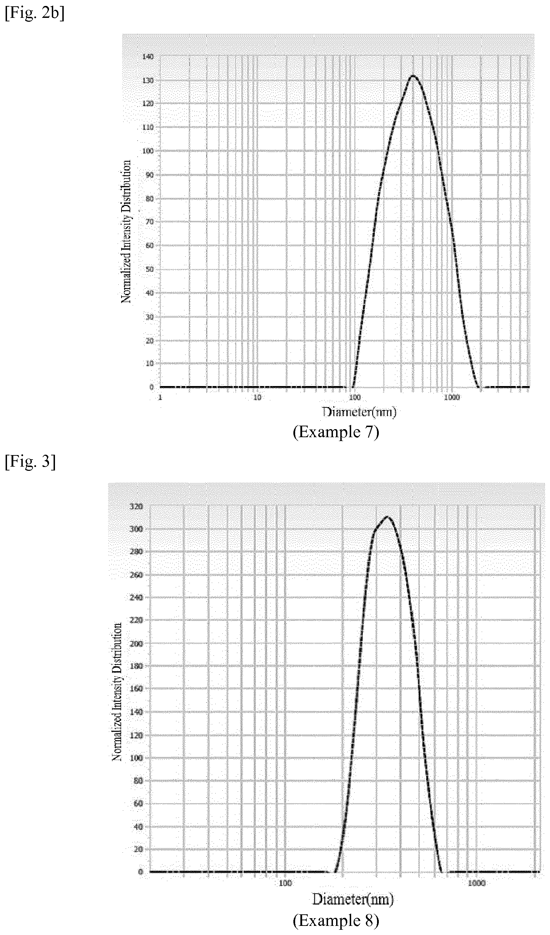

The obtained powder was dispersed in demineralized distilled water at a concentration of 1 mg/ml (based on Nilotinib), and then the particle size was measured with ELSZ-1000 (Otsuka) in DLS (Dynamic light scattering) manner. The particle size distributions of the nanoparticles prepared in Examples 6 and 7 are shown in Figure 2.

**[Table 2]**

| Example | Active ingredient | Lipid (LA) | PVP k30 | SLS | Roll mill conditions | | | Mean particle size (nm) | Particle size distribution (PDI) |
|---|---|---|---|---|---|---|---|---|---|
| | | | | | R.S (rpm) | R.T (°C) | O.N (number) | | |
| 6 | 1 g | 0.3 g | 0.3 g | 0.05 g | 60 | 27 to 30 | 25 | 179.8 | 0.183 |
| 7 | 1 g | 0.5 g | 0.3 g | 0.05 g | 60 | 27 to 30 | 70 | 325.7 | 0.262 |

| | | | | | | | | | |
|---|---|---|---|---|---|---|---|---|---|
| Active ingredient: Nilotinib | | | | | | | | | |

### Example 8

As an active ingredient, 10 g of Erlotinib (free base) was added into a beaker together with 20 g of MA as a lubricant and 20 g of DMSO as water miscible solvent, and heated to 40°C for complete dissolution. Then, the solution was poured into 500 ml of water at 10°C to prepare a solid dispersion wherein Erlotinib and MA were mixed well, and the solid dispersion was filtered and dried under reduced pressure. To 30 g of the dried solid mixture, 3 g of hydroxypropyl cellulose (HPC; ssL) and 1 g of sucrose stearate (s-1670) were added together with 15 ml of demineralized distilled water, and roll mill was performed several times to mix them well. To the resulting mixture, continuous 40 times milling was performed using 3-roll mill with the roller speed (R.S) of 60 rpm and roller temperature (R.T) of 25 to 30°C. The water used in mixing was all evaporated during the milling process, and the residual water content was 1%(w/w) or less.

The obtained solid dispersion type mixture was placed in a high pressure reactor, and carbon dioxide as supercritical fluid was continuously added thereto at 15 to 25°C under 7.1 MPa to 10.1 MPa (70 to 100 atmospheres) to remove the lipid used as a lubricant, and a mixture powder, wherein Erlotinib, HPC ssL, and sucrose stearate were mixed, was obtained.

The obtained powder was dispersed in demineralized distilled water at a concentration of 1 mg/ml (based on Erlotinib), and then the particle size was measured as the mean particle size of 341.7 nm with ELSZ-1000 (Otsuka) in DLS (Dynamic light scattering) manner. The particle size distribution of the nanoparticles prepared in Example 8 is shown in Figure 3.

### Example 9

A separate experiment confirmed that in case of dissolving and recrystallizing Sorafenib tosylate III in a solvent, there was a problem of crystal form change and partial generation of free base compound by loss of tosylate salt.

Accordingly, 3 g of Sorafenib tosylate III powder was mixed with 9 g of MA powder as a lubricant well, and thereto, 0.75 g of hydroxypropyl methylcellulose (HPMC; 5cp), 0.3 g of PVP (k30), and 0.09 g of poloxamer (407) were added together with 2.25 ml of demineralized distilled water, and roll mill was performed several times to mix them well. To the resulting mixture, continuous 8 times milling was performed using 3-roll mill with the roller speed (R.S) of 60 rpm and roller temperature (R.T) of 15 to 19°C. At this time, the residual water content (measured by Karl Fischer method) was 9%(w/w), and the mixture was dried under reduced pressure at room temperature (25°C). The obtained solid dispersion type mixture was placed in a high pressure reactor, and carbon dioxide as supercritical fluid was continuously added thereto at 15 to 25°C under 7.1 MPa to 10.1 MPa (70 to 100 atmospheres) to remove the lipid used as a lubricant, and a mixture powder, wherein Sorafenib, HPMC, PVP, and poloxamer were mixed, was obtained.

The obtained powder was dispersed in demineralized distilled water at a concentration of 1 mg/ml (based on Sorafenib), and then the particle size was measured as the mean particle size of 496.8 nm with ELSZ-1000 (Otsuka) in DLS (Dynamic light scattering) manner.

### Example 10

3 g of Sorafenib tosylate III powder was mixed with 4.5 g of MA powder as a lubricant well, and thereto, 0.3 g of HPC (ssL), 0.3 g of polyoxyethylene 40 stearate (PS), 0.3 g of PVP (k30), 0.3 g of sucrose stearate (SS; s-1670), and 0.9 g of poloxamer (407) were added together with 4.5 ml of demineralized distilled water and mixed well. To the resulting mixture, continuous 40 times milling was performed using 3-roll mill with the roller speed (R.S) of 60 rpm and roller temperature (R.T) of 15 to 19°C. At this time, the residual water content (measured by Karl Fischer method) was 5%(w/w), and the mixture was dried under reduced pressure at room temperature (25°C). The obtained solid dispersion type mixture was placed in a high pressure reactor, and carbon dioxide as supercritical fluid was continuously added thereto at 15 to 25°C under 7.1 MPa to 10.1 MPa (70 to 100 atmospheres) to remove the lipid used as a lubricant, and a mixture powder, wherein Sorafenib, HPC, PVP, PS, SS, and poloxamer were mixed, was obtained.

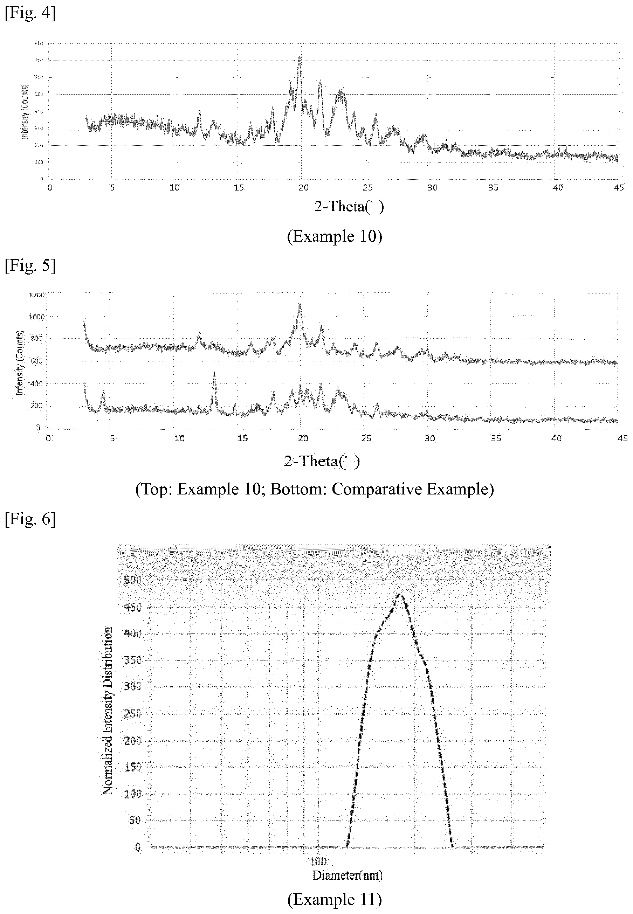

The obtained powder was dispersed in demineralized distilled water at a concentration of 1 mg/ml (based on Sorafenib), and then the particle size was measured as the mean particle size of 446.0 nm with ELSZ-1000 (Otsuka) in DLS (Dynamic light scattering) manner. In addition, it was confirmed through pXRD analysis that the crystal form of Sorafenib tosylate III was maintained. The pXRD analysis result of the nanoparticles prepared in Example 10 is shown in Figure 4.

### Comparative Example

Except that MA as a lubricant was not added, the method of Example 10 was conducted with the same addition ratios of all excipients and the same process conditions of milling. The milling process took much time since the lipid as a lubricant was not added and thus very heavy load was applied. In addition, since no lubricant was added, the process for removing lubricant by using carbon dioxide was not performed. After performing the milling only, the mixture was dried under reduced pressure. The obtained mixture powder, wherein Sorafenib, HPC, PVP, PS, SS and poloxamer were mixed, was dispersed in demineralized distilled water at a concentration of 1 mg/ml (based on Sorafenib), and then the particle size was measured as the mean particle size of 786.0 nm with ELSZ-1000 (Otsuka) in DLS (Dynamic light scattering) manner. In addition, it was confirmed through pXRD analysis that the crystal form of Sorafenib tosylate was changed to type I. The pXRD analysis results of the nanoparticles prepared in Example 10 and Comparative Example in comparison are shown in Figure 5.

### Example 11

50 g of Sorafenib (free base) and 100 g of MA as a lubricant were added to 100 ml of DMF, and heated to 40°C for complete dissolution. Then, the solution was poured into cool water at 20°C for solidification, and the resulting mixture was agitated at room temperature for about 3 hours, and filtered and dried under reduced pressure to obtain a mixture of Sorafenib and MA. To 9 g of this mixture, 0.3 g of HPC, 0.45 g of PVP (k30) and 0.45 g of poloxamer (407) were added together with 3 ml of demineralized water, and roll mill was performed several times to mix them well. To the resulting mixture, continuous 50 times milling was performed using 3-roll mill with the roller speed (R.S) of 60 rpm and roller temperature (R.T) of 27 to 30°C. The water used in mixing was all evaporated during the milling process, and the residual water content was 1%(w/w) or less. The obtained solid dispersion type mixture was placed in a high pressure reactor, and carbon dioxide as supercritical fluid was continuously added thereto at 15 to 25°C under 7.1 MPa to 10.1 MPa (70 to 100 atmospheres) to remove the lipid used as a lubricant, and a mixture powder, wherein Sorafenib, HPC, PVP (k30), and poloxamer (407) were mixed, was obtained.

The obtained powder was dispersed in demineralized distilled water at a concentration of 1 mg/ml (based on Sorafenib), and then the particle size was measured as the mean particle size of 196.5 nm with ELSZ-1000 (Otsuka) in DLS (Dynamic light scattering) manner. The particle size distribution of the nanoparticles prepared in Example 11 is shown in Figure 6.

### Example 12

As an active ingredient, 40 g of Posaconazole (antifungal agent) and 80 g of MA as a lubricant were added to 120 ml of DMSO, and heated to 40°C for complete dissolution. Then, the solution was poured into water at 30°C for solidification, and the resulting mixture was agitated for about 3 hours, and filtered and dried under reduced pressure to obtain a mixture of Posaconazole and lipid. To 15 g of this mixture, 1.5 g of HPC, 0.5 g of poloxamer (407) and 0.5 g of PVP (k30) were added together with 5 ml of demineralized water, and roll mill was performed several times at a temperature of 20°C or lower to mix them uniformly. At this time, the residual water content (measured by Karl Fischer method) was 21%(w/w), and the mixture was dried under reduced pressure at room temperature (25°C). The obtained solid dispersion type mixture was placed in a high pressure reactor, and carbon dioxide as supercritical fluid was continuously added thereto at 15 to 25°C under 7.1 MPa to 10.1 MPa (70 to 100 atmospheres) to remove the lipid used as a lubricant, and a mixture powder, wherein Posaconazole, HPC, PVP (k30) and poloxamer (407) were mixed, was obtained.

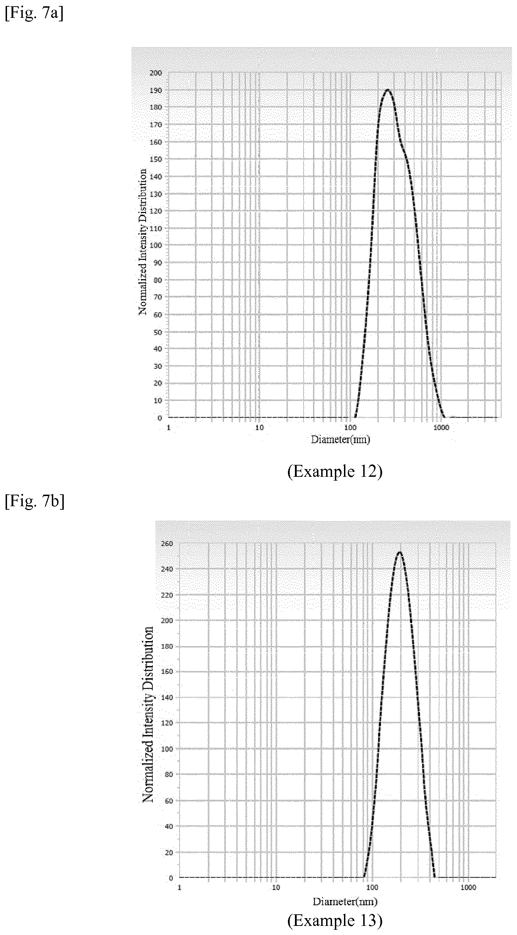

The obtained powder was dispersed in demineralized distilled water at a concentration of 1 mg/ml (based on Posaconazole), and then the particle size was measured as the mean particle size of 286.5 nm with ELSZ-1000 (Otsuka) in DLS (Dynamic light scattering) manner. The particle size distribution of the nanoparticles prepared in Example 12 is shown in Figure 7.

### Example 13

To 15 g of the mixture of Posaconazole and MA as a lubricant prepared in Example 12, 1.5 g of HPC (ssL), 0.5 g of poloxamer (407) and 0.5 g of PVP (k30) were added together with 5 ml of demineralized water, and roll mill was performed several times at a temperature of 20°C or lower to mix them uniformly. To the resulting mixture, continuous 20 times milling was performed using 3-roll mill with the roller speed (R.S) of 60 rpm and roller temperature (R.T) of 13 to 17°C. At this time, the residual water content (measured by Karl Fischer method) was 8.9%(w/w), and the mixture was dried under reduced pressure at room temperature (25°C). The obtained solid dispersion type mixture was placed in a high pressure reactor, and carbon dioxide as supercritical fluid was continuously added thereto at 15 to 25°C under 7.1 MPa to 10.1 MPa (70 to 100 atmospheres) to remove the lipid used as a lubricant, and a mixture powder, wherein Posaconazole, HPC, PVP (k30), and poloxamer (407) were mixed, was obtained.

The obtained powder was dispersed in demineralized distilled water at a concentration of 1 mg/ml (based on Posaconazole), and then the particle size was measured as the mean particle size of 185.9 nm with ELSZ-1000 (Otsuka) in DLS (Dynamic light scattering) manner. The particle size distribution of the nanoparticles prepared in Example 13 is shown in Figure 7.

### Example 14

Except that 2-roll mill was used, the method of Example 13 was conducted with the same addition ratios of all excipients to the active ingredient and the same temperature condition of milling process and number of milling operation, to obtain a mixture powder wherein Posaconazole, HPC, PVP (k30) and poloxamer (407) were mixed. From this mixture powder, the lipid as a lubricant was removed by the same method as in Example 13 to prepare nanoparticles having the mean particle size of 199.2 nm.

### Examples 15 and 16

As an active ingredient, 1 part by weight of Posaconazole and 0.5 part by weight or 1 part by weight of MA as a lubricant were added to 2 parts by weight of DMSO, and heated to 45°C for complete dissolution. Then, the solution was poured into demineralized water at 25°C for solidification. After agitation for 3 hours, and the resulting mixture was filtered and dried under reduced pressure to obtain solid mixtures of Posaconazole:MA of 1:0.5 and 1:1, respectively.

To each mixture, based on 1 part by weight of Posaconazole, 0.3 part by weight of HPC, 0.1 part by weight of poloxamer (407) and 0.1 part by weight of PVP (k90) were added together with 1 part by weight of demineralized water, and roll mill was performed several times at a temperature of 20°C or lower to mix them uniformly. To the resulting mixtures, continuous 45 times and 60 times milling were performed, respectively, using 3-roll mill with the roller speed (R.S) of 60 rpm and roller temperature (R.T) of 15 to 17°C. At this time, the residual water contents (measured by Karl Fischer method) were 8.04%(w/w) and 7.61%(w/w), respectively, and the mixtures were dried under reduced pressure at room temperature (25°C). The obtained solid dispersion type mixture was placed in a high pressure reactor, and carbon dioxide as supercritical fluid was continuously added thereto at 15 to 25°C under 7.1 MPa to 10.1 MPa (70 to 100 atmospheres) to remove the lipid used as a lubricant and obtain a mixture powder, wherein Posaconazole, HPC, PVP (k90) and poloxamer (407) were mixed.

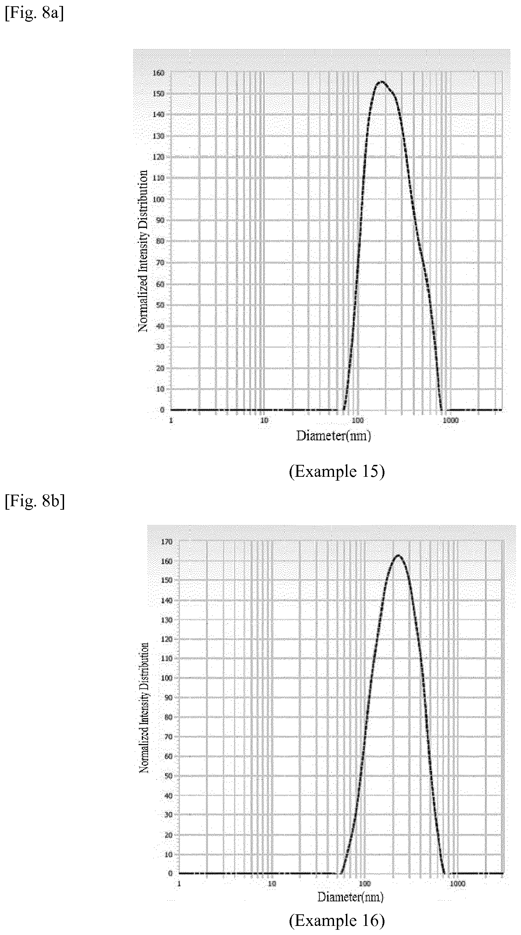

The obtained powder was dispersed in demineralized distilled water at a concentration of 1 mg/ml (based on Posaconazole), and then the particle size was measured with ELSZ-1000 (Otsuka) in DLS (Dynamic light scattering) manner. The mean particle size is shown in the following Table 3. The particle size distributions of the nanoparticles prepared in Examples 15 and 16 are shown in Figure 8.

**[Table 3]**

| Example | Active ingredient | Lipid (MA) | Roll mill conditions | | | Residual water content (%) | Mean particle size (nm) |
|---|---|---|---|---|---|---|---|
| | | | R.S (rpm) | R.T (°C) | O.N (number) | | |
| 15 | 1 g | 0.5 g | 60 | 15 to 17 | 45 | 8.04 | 211.0 |
| 16 | 1 g | 1 g | 60 | 15 to 17 | 60 | 7.61 | 191.8 |

| | | | | | | | |
|---|---|---|---|---|---|---|---|
| Active ingredient: Posaconazole | | | | | | | |

### Examples 17 to 19

As an active ingredient, 6 g of Posaconazole, 12 g of MA as a lubricant and 1.8 g of HPC (ssL) were mixed together with 6 ml of demineralized water, and to the resulting mixture, continuous 30 times, 50 times and 70 times milling were performed, respectively, using 3-roll mill with the roller speed (R.S) of 60 rpm and roller temperature (R.T) of 27 to 30°C. The water used in mixing was all evaporated during the milling process, and the residual water content was 1%(w/w) or less. The obtained solid dispersion type mixture was placed in a high pressure reactor, and carbon dioxide as supercritical fluid was continuously added thereto at 15 to 25°C under 7.1 MPa to 10.1 MPa (70 to 100 atmospheres) to remove the lipid used as a lubricant, and a mixture powder, wherein Posaconazole and HPC (ssL) were mixed, was obtained.

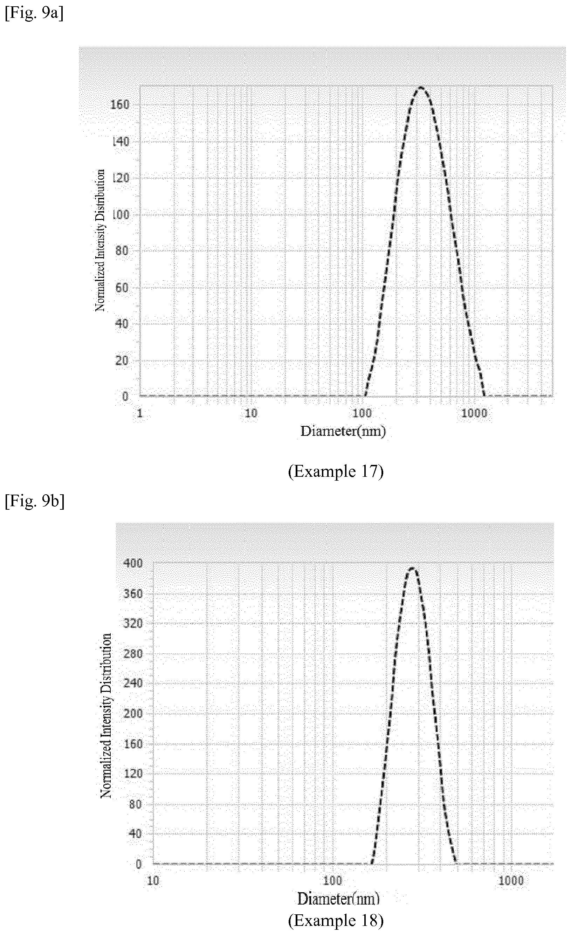

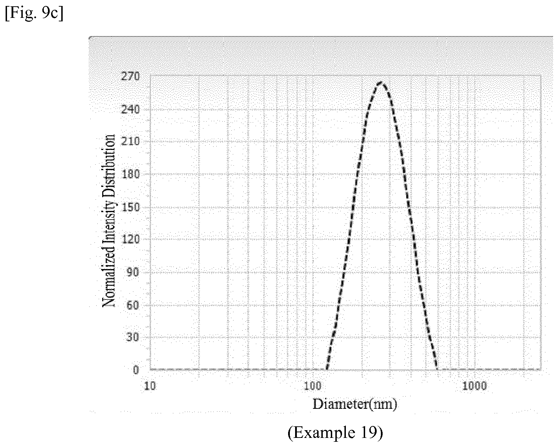

The obtained powder was dispersed in demineralized distilled water at a concentration of 1 mg/ml (based on Posaconazole), and then the particle size was measured with ELSZ-1000 (Otsuka) in DLS (Dynamic light scattering) manner. The mean particle size is shown in the following Table 4. The particle size distributions of the nanoparticles prepared in Examples 17 to 19 are shown in Figure 9.

**[Table 3]**

| Example | Active ingredient | Lipid (MA) | Roll mill conditions | | | Mean particle size (nm) |
|---|---|---|---|---|---|---|
| | | | R.S (rpm) | R.T (°C) | O.N (number) | |
| 17 | 6 g | 18 g | 60 | 27 to 30 | 30 | 314.2 |
| 18 | 6 g | 18 g | 60 | 27 to 30 | 50 | 274.6 |
| 19 | 6 g | 18 g | 60 | 27 to 30 | 70 | 253.1 |

| | | | | | | |
|---|---|---|---|---|---|---|
| Active ingredient: Posaconazole | | | | | | |

### Example 20

As an active ingredient, 6 g of Posaconazole, 12 g of MA as a lubricant and 1.8 g of HPC (ssL) were mixed together with 6 ml of demineralized water, and to the resulting mixture, continuous 60 times milling was performed using 3-roll mill with the roller speed (R.S) of 60 rpm and roller temperature (R.T) of 27 to 30°C. The water used in mixing was all evaporated during the milling process, and the residual water content was 1%(w/w) or less. To the obtained solid dispersion, 0.6 g of poloxamer (407) and 0.6 g of PVP (k30) were added together with 6 ml of demineralized water, and roll mill was performed several times at a temperature of 20°C or lower to mix them uniformly. To the resulting mixture, continuous 24 times milling was performed using 3-roll mill with the roller speed (R.S) of 60 rpm and roller temperature (R.T) of 13 to 15°C. At this time, the residual water content (measured by Karl Fischer method) was 8.48%(w/w), and the mixtures were dried under reduced pressure at room temperature (25°C). The obtained solid dispersion type mixture was placed in a high pressure reactor, and carbon dioxide as supercritical fluid was continuously added thereto at 15 to 25°C under 7.1 MPa to 10.1 MPa (70 to 100 atmospheres) to remove the lipid used as a lubricant, and a mixture powder, wherein Posaconazole, HPC, PVP (k30) and poloxamer (407) were mixed, was obtained.

The obtained powder was dispersed in demineralized distilled water at a concentration of 1 mg/ml (based on Posaconazole), and then the particle size was measured as the mean particle size of 254.4 nm with ELSZ-1000 (Otsuka) in DLS (Dynamic light scattering) manner.

## Claims

1. A method for preparing nanoparticle of active ingredient comprising:
(1) a step of providing a mixture comprising the active ingredient, a lipid as a lubricant, and a biocompatible polymer having a glass transition temperature of 80°C or higher;
(2) a step of pulverizing the resulting product of said step (1) through milling process; and
(3) a step of removing the lipid from the resulting product of said step (2) by using supercritical fluid, wherein the lipid is used in an amount of five parts by weight or less, based on one part by weight of the active ingredient.

2. The method for preparing nanoparticle of active ingredient according to claim 1, wherein said step (1) is a step of physically and uniformly mixing the active ingredient, a lipid as a lubricant, and a biocompatible polymer having a glass transition temperature of 80°C or higher.

3. The method for preparing nanoparticle of active ingredient according to claim 1, wherein said step (1) is a step of mixing the active ingredient and a lipid as a lubricant; and to this mixture, a biocompatible polymer having a glass transition temperature of 80°C or higher is added together with demineralized water; and then physically mixing the resulting product uniformly.

4. The method for preparing nanoparticle of active ingredient according to claim 1, wherein said step (1) is a step of physically and uniformly mixing the active ingredient, a lipid as a lubricant, a biocompatible polymer having a glass transition temperature of 80°C or higher, and one or more additional components selected from a biocompatible polymer having a glass transition temperature of lower than 80°C, a surfactant, and an anti-coagulation agent.

5. The method for preparing nanoparticle of active ingredient according to claim 1, wherein said step (1) is a step of mixing the active ingredient and a lipid as a lubricant; and to this mixture, a biocompatible polymer having a glass transition temperature of 80°C or higher and one or more additional components selected from a biocompatible polymer having a glass transition temperature of lower than 80°C, a surfactant, and an anti-coagulation agent are added together with demineralized water; and then physically mixing the resulting product uniformly.

6. The method for preparing nanoparticle of active ingredient according to claim 1, wherein said step (1) is a step of physically and uniformly mixing a solidified mixture and a biocompatible polymer having a glass transition temperature of 80°C or higher, wherein the solidified mixture was prepared by pouring a solution, where the active ingredient and a lipid as a lubricant are dissolved in water miscible organic solvent, into water for solidification; filtering and drying the mixture.

7. The method for preparing nanoparticle of active ingredient according to claim 1, wherein said step (1) is a step of physically and uniformly mixing a solidified mixture and a biocompatible polymer having a glass transition temperature of 80°C or higher with demineralized water, wherein the solidified mixture was prepared by pouring a solution, where the active ingredient and a lipid as a lubricant are dissolved in water miscible organic solvent, into water for solidification; filtering and drying the mixture.

8. The method for preparing nanoparticle of active ingredient according to claim 1, wherein said step (1) is a step of physically and uniformly mixing a solidified mixture, a biocompatible polymer having a glass transition temperature of 80°C or higher, and one or more additional components selected from a biocompatible polymer having a glass transition temperature of lower than 80°C, a surfactant, and an anti-coagulation agent, wherein the solidified mixture was prepared by pouring a solution, where the active ingredient and a lipid as a lubricant are dissolved in water miscible organic solvent, into water for solidification; filtering and drying the mixture.

9. The method for preparing nanoparticle of active ingredient according to claim 1, wherein said step (1) is a step of physically and uniformly mixing a solidified mixture, a biocompatible polymer having a glass transition temperature of 80°C or higher, and one or more additional components selected from a biocompatible polymer having a glass transition temperature of lower than 80°C, a surfactant, and an anti-coagulation agent together with demineralized water, wherein the solidified mixture was prepared by pouring a solution, where the active ingredient and a lipid as a lubricant are dissolved in water miscible organic solvent, into water for solidification; filtering and drying the mixture.

10. The method for preparing nanoparticle of active ingredient according to claim 1, wherein said step (1) is a step of preparing a solidified mixture by pouring a solution, where the active ingredient, a lipid as a lubricant and a biocompatible polymer having a glass transition temperature of 80°C or higher are dissolved in water miscible organic solvent, into water for solidification; and filtering and drying the mixture.

11. The method for preparing nanoparticle of active ingredient according to claim 1, wherein said step (1) is a step of physically and uniformly mixing a solidified mixture and one or more additional components selected from a biocompatible polymer having a glass transition temperature of lower than 80°C, a surfactant, and an anti-coagulation agent, wherein the solidified mixture was prepared by pouring a solution, where the active ingredient, a lipid as a lubricant, and a biocompatible polymer having a glass transition temperature of 80°C or higher are dissolved in water miscible organic solvent, into water for solidification; filtering and drying the mixture.

12. The method for preparing nanoparticle of active ingredient according to claim 1, wherein said step (1) is a step of physically and uniformly mixing a solidified mixture and one or more additional components selected from a biocompatible polymer having a glass transition temperature of lower than 80°C, a surfactant, and an anti-coagulation agent together with demineralized water, wherein the solidified mixture was prepared by pouring a solution, where the active ingredient, a lipid as a lubricant, and a biocompatible polymer having a glass transition temperature of 80°C or higher are dissolved in water miscible organic solvent, into water for solidification; filtering and drying the mixture.

13. The method for preparing nanoparticle of active ingredient according to claim 1, wherein the milling process in said step (2) is performed continuously by using counter rotating rolls.

14. The method for preparing nanoparticle of active ingredient according to claim 1, wherein the lipid removal in said step (3) is performed by continuously adding supercritical fluid into a reactor containing the resulting product of said step (2) and discharging it therefrom.

15. The method for preparing nanoparticle of active ingredient according to claim 1, wherein the lipid removal using supercritical fluid in said step (3) is performed under a pressure condition of 50 atmospheres or higher and a temperature condition of 5 to 60°C.

16. The method for preparing nanoparticle of active ingredient according to any one of claims 1 to 15, wherein the active ingredient is selected from organic compounds, organometallic compounds, natural extracts, proteins, and combinations thereof.

17. The method for preparing nanoparticle of active ingredient according to any one of claims 1 to 15, wherein the lipid is selected from saturated fatty acids having 10 to 22 carbon atoms, esters of saturated fatty acid having 10 to 22 carbon atoms, saturated fatty alcohols having 10 to 22 carbon atoms, mono-, di or tri-glycerides having saturated fatty acid group having 10 to 22 carbon atoms, hydrocarbons having 14 or more carbon atoms, and combinations thereof.

18. The method for preparing nanoparticle of active ingredient according to any one of claims 1 to 15, wherein the biocompatible polymer having a glass transition temperature (Tg) of 80°C or higher is one or more selected from cellulose-based biocompatible polymer having a Tg of 80°C or higher, polyvinyl pyrrolidone having a Tg of 80°C or higher, polyvinyl alcohol having a Tg of 80°C or higher, and Eudragit-based biocompatible polymer having a Tg of 80°C or higher.

## Patentansprüche

1. Verfahren zur Herstellung von Aktivmaterial-Nanopartikeln umfassend:
(1) einen Schritt des Bereitstellens einer Mischung umfassend das Aktivmaterial, ein Lipid als Schmiermittel und ein biokompatibles Polymer mit einer Glasübergangstemperatur von 80 °C oder höher;
(2) einen Schritt des Zerkleinerns des resultierenden Produkts aus Schritt (1) durch einen Mahlprozess; und
(3) einen Schritt des Entfernens des Lipids aus dem resultierenden Produkt des Schritts (2) unter Verwendung eines überkritischen Fluids, wobei das Lipid in einer Menge von fünf Gewichtsteilen oder weniger, bezogen auf einen Gewichtsteil des Aktivmaterials, verwendet wird.

2. Verfahren zur Herstellung von Aktivmaterial-Nanopartikeln gemäß Anspruch 1, wobei der Schritt (1) ein Schritt des physikalischen und gleichmäßigen Mischens des Aktivmaterials, eines Lipids als Schmiermittel und eines biokompatiblen Polymers mit einer Glasübergangstemperatur von 80 °C oder höher ist.

3. Verfahren zur Herstellung von Aktivmaterial-Nanopartikeln gemäß Anspruch 1, wobei der Schritt (1) ein Schritt des Mischens des Aktivmaterials und eines Lipids als Schmiermittel ist; und zu dieser Mischung wird ein biokompatibles Polymer mit einer Glasübergangstemperatur von 80 °C oder höher zusammen mit demineralisiertem Wasser hinzugefügt; und dann wird das resultierende Produkt physikalisch gleichmäßig gemischt.

4. Das Verfahren zur Herstellung von Aktivmaterial-Nanopartikeln gemäß Anspruch 1, wobei der Schritt (1) ein Schritt des physikalischen und gleichmäßigen Mischens des Aktivmaterials, eines Lipids als Schmiermittel, eines biokompatiblen Polymers mit einer Glasübergangstemperatur von 80 °C oder höher und einer oder mehrerer zusätzlicher Komponenten, ausgewählt aus einem biokompatiblen Polymer mit einer Glasübergangstemperatur von weniger als 80 °C, einem Tensid und einem Antikoagulationsmittel, ist.

5. Verfahren zur Herstellung von Aktivmaterial-Nanopartikeln gemäß Anspruch 1, wobei der Schritt (1) ein Schritt des Mischens des Aktivmaterials und eines Lipids als Schmiermittel ist; und zu dieser Mischung ein biokompatibles Polymer mit einer Glasübergangstemperatur von 80 °C oder höher und eine oder mehrere zusätzliche Komponenten, ausgewählt aus einem biokompatiblen Polymer mit einer Glasübergangstemperatur von weniger als 80 °C, einem Tensid und einem Antikoagulationsmittel, zusammen mit demineralisiertem Wasser hinzugefügt werden; und dann das resultierende Produkt gleichmäßig physikalisch gemischt wird.

6. Verfahren zur Herstellung von Aktivmaterial-Nanopartikeln gemäß Anspruch 1, wobei der Schritt (1) ein Schritt des physikalischen und gleichmäßigen Mischens eines verfestigten Gemischs und eines biokompatiblen Polymers mit einer Glasübergangstemperatur von 80 °C oder höher ist, wobei das verfestigte Gemisch durch Gießen einer Lösung, in der das Aktivmaterial und ein Lipid als Schmiermittel in einem mit Wasser mischbaren organischen Lösungsmittel gelöst sind, in Wasser zur Verfestigung, Filtrieren und Trocknen des Gemischs, hergestellt wurde.

7. Verfahren zur Herstellung von Aktivmaterial-Nanopartikeln gemäß Anspruch 1, wobei der Schritt (1) ein Schritt des physikalischen und gleichmäßigen Vermischens eines verfestigten Gemischs und eines biokompatiblen Polymers mit einer Glasübergangstemperatur von 80 °C oder höher mit demineralisiertem Wasser ist, wobei das verfestigte Gemisch durch Gießen einer Lösung, in der das Aktivmaterial und ein Lipid als Schmiermittel in einem mit Wasser mischbaren organischen Lösungsmittel gelöst sind, in Wasser zur Verfestigung, Filtrieren und Trocknen des Gemischs, hergestellt wurde.

8. Verfahren zur Herstellung von Aktivmaterial-Nanopartikeln gemäß Anspruch 1, wobei der Schritt (1) ein Schritt des physikalischen und gleichmäßigen Mischens eines verfestigten Gemischs, eines biokompatiblen Polymers mit einer Glasübergangstemperatur von 80 °C oder höher und einer oder mehrerer zusätzlicher Komponenten, ausgewählt aus einem biokompatiblen Polymer mit einer Glasübergangstemperatur von weniger als 80 °C, einem Tensid und einem Antikoagulationsmittel, wobei die verfestigte Mischung durch Gießen einer Lösung, in der das Aktivmaterial und ein Lipid als Schmiermittel in einem mit Wasser mischbaren organischen Lösungsmittel gelöst sind, in Wasser zur Verfestigung, Filtrieren und Trocknen des Gemischs, hergestellt wurde.

9. Verfahren zur Herstellung von Aktivmaterial-Nanopartikeln gemäß Anspruch 1, wobei der Schritt (1) ein Schritt des physikalischen und gleichmäßigen Mischens eines verfestigten Gemischs, eines biokompatiblen Polymers mit einer Glasübergangstemperatur von 80 °C oder höher und einer oder mehrerer zusätzlicher Komponenten, ausgewählt aus einem biokompatiblen Polymer mit einer Glasübergangstemperatur von weniger als 80 °C einem Tensid und einem Antikoagulationsmittel zusammen mit demineralisiertem Wasser, wobei das verfestigte Gemisch durch Gießen einer Lösung, in der das Aktivmaterial und ein Lipid als Schmiermittel in einem mit Wasser mischbaren organischen Lösungsmittel gelöst sind, in Wasser zur Verfestigung, Filtrieren und Trocknen des Gemisches, hergestellt wurde.

10. Verfahren zur Herstellung von Aktivmaterial-Nanopartikeln gemäß Anspruch 1, wobei der Schritt (1) ein Schritt der Herstellung eines verfestigten Gemischs ist, indem eine Lösung, in der das Aktivmaterial, ein Lipid als Schmiermittel und ein biokompatibles Polymer mit einer Glasübergangstemperatur von 80 °C oder höher in einem mit Wasser mischbaren organischen Lösungsmittel gelöst sind, zur Verfestigung in Wasser gegossen wird; und Filtrieren und Trocknen des Gemischs.

11. Verfahren zur Herstellung von Aktivmaterial-Nanopartikeln gemäß Anspruch 1, wobei der Schritt (1) ein Schritt des physikalischen und gleichmäßigen Mischens eines verfestigten Gemisches und einer oder mehrerer zusätzlicher Komponenten ist, ausgewählt aus einem biokompatiblen Polymer mit einer Glasübergangstemperatur von weniger als 80 °C, einem Tensid und einem Antikoagulationsmittel, wobei das verfestigte Gemisch durch Gießen einer Lösung, in der das Aktivmaterial, ein Lipid als Schmiermittel und ein biokompatibles Polymer mit einer Glasübergangstemperatur von 80 °C oder höher in einem mit Wasser mischbaren organischen Lösungsmittel gelöst sind, in Wasser zur Verfestigung, Filtrieren und Trocknen des Gemisches, hergestellt wurde.

12. Verfahren zur Herstellung von Aktivmaterial-Nanopartikeln gemäß Anspruch 1, wobei der Schritt (1) ein Schritt des physikalischen und gleichmäßigen Mischens eines verfestigten Gemisches und einer oder mehrerer zusätzlicher Komponenten ausgewählt aus einem biokompatiblen Polymer mit einer Glasübergangstemperatur von weniger als 80 °C, einem Tensid und einem Antikoagulationsmittel zusammen mit demineralisiertem Wasser, wobei das verfestigte Gemisch durch Gießen einer Lösung, in der das Aktivmaterial, ein Lipid als Schmiermittel und ein biokompatibles Polymer mit einer Glasübergangstemperatur von 80 °C oder höher in einem mit Wasser mischbaren organischen Lösungsmittel gelöst sind, in Wasser zur Verfestigung, Filtrieren und Trocknen des Gemisches, hergestellt wurde.

13. Verfahren zur Herstellung von Aktivmaterial-Nanopartikeln gemäß Anspruch 1, wobei der Mahlprozess in Schritt (2) kontinuierlich unter Verwendung gegenläufiger Walzen durchgeführt wird.

14. Verfahren zur Herstellung von Aktivmaterial-Nanopartikeln gemäß Anspruch 1, wobei die Entfernung des Lipids in Schritt (3) durch kontinuierliche Zugabe von überkritischem Fluid in einen Reaktor, der das resultierende Produkt aus Schritt (2) enthält, und dessen Entleeren daraus durchgeführt wird.

15. Verfahren zur Herstellung von Aktivmaterial-Nanopartikeln gemäß Anspruch 1, wobei die Entfernung des Lipids unter Verwendung eines überkritischen Fluids in Schritt (3) unter einer Druckbedingung von 50 Atmosphären oder höher und einer Temperaturbedingung von 5 bis 60 °C durchgeführt wird.

16. Verfahren zur Herstellung von Aktivmaterial-Nanopartikeln gemäß einem der Ansprüche 1 bis 15, wobei das Aktivmaterial ausgewählt ist aus organischen Verbindungen, organometallischen Verbindungen, natürlichen Extrakten, Proteinen und Kombinationen davon.

17. Verfahren zur Herstellung von Aktivmaterial-Nanopartikeln gemäß einem der Ansprüche 1 bis 15, wobei das Lipid ausgewählt ist aus gesättigten Fettsäuren mit 10 bis 22 Kohlenstoffatomen, Estern von gesättigten Fettsäuren mit 10 bis 22 Kohlenstoffatomen, gesättigten Fettalkoholen mit 10 bis 22 Kohlenstoffatomen, Mono-, Di- oder Triglyceriden mit gesättigten Fettsäuregruppen mit 10 bis 22 Kohlenstoffatomen, Kohlenwasserstoffen mit 14 oder mehr Kohlenstoffatomen und Kombinationen davon.

18. Verfahren zur Herstellung von Aktivmaterial-Nanopartikeln gemäß einem der Ansprüche 1 bis 15, wobei das biokompatible Polymer mit einer Glasübergangstemperatur (Tg) von 80 °C oder höher eines oder mehrere ausgewählt ist/sind aus biokompatiblem Polymer auf Cellulosebasis mit einer Tg von 80 °C oder höher, Polyvinylpyrrolidon mit einer Tg von 80 °C oder höher, Polyvinylalkohol mit einer Tg von 80 °C oder höher und biokompatiblem Polymer auf Eudragitbasis mit einer Tg von 80 °C oder höher.

## Revendications

1. Un procédé de préparation de nanoparticules de matériau actif comprenant :
(1) une étape de fourniture d'un mélange comprenant le matériau actif, un lipide en tant que lubrifiant, et un polymère biocompatible ayant une température de transition vitreuse de 80°C ou plus ;
(2) une étape de pulvérisation du produit résultant de ladite étape (1) par un processus de broyage ; et
(3) une étape de retrait du lipide du produit résultant de ladite étape (2) en utilisant un fluide supercritique, dans lequel le lipide est utilisé en une quantité de cinq parties en poids ou moins, sur la base d'une partie en poids du matériau actif.

2. Le procédé de préparation de nanoparticules de matériau actif selon la revendication 1, dans lequel ladite étape (1) est une étape de mélange physique et uniforme du matériau actif, d'un lipide en tant que lubrifiant, et d'un polymère biocompatible ayant une température de transition vitreuse de 80°C ou plus.

3. Le procédé de préparation de nanoparticules de matériau actif selon la revendication 1, dans lequel ladite étape (1) est une étape de mélange du matériau actif et d'un lipide en tant que lubrifiant ; et à ce mélange, un polymère biocompatible ayant une température de transition vitreuse de 80°C ou plus est ajouté avec de l'eau déminéralisée ; puis le produit résultant est physiquement mélangé uniformément.

4. Le procédé de préparation de nanoparticules de matériau actif selon la revendication 1, dans lequel ladite étape (1) est une étape de mélange physique et uniforme du matériau actif, d'un lipide en tant que lubrifiant, d'un polymère biocompatible ayant une température de transition vitreuse de 80°C ou plus, et d'un ou plusieurs composants supplémentaires choisis parmi un polymère biocompatible ayant une température de transition vitreuse inférieure à 80°C, un tensioactif, et un agent anti-coagulation.

5. Le procédé de préparation de nanoparticules de matériau actif selon la revendication 1, dans lequel ladite étape (1) est une étape de mélange du matériau actif et d'un lipide en tant que lubrifiant ; et à ce mélange, un polymère biocompatible ayant une température de transition vitreuse de 80°C ou plus et un ou plusieurs composants supplémentaires choisis parmi un polymère biocompatible ayant une température de transition vitreuse inférieure à 80°C, un tensioactif, et un agent anti-coagulation sont ajoutés en même temps que de l'eau déminéralisée ; puis le produit résultant est physiquement mélangé uniformément.

6. Le procédé de préparation de nanoparticules de matériau actif selon la revendication 1, dans lequel ladite étape (1) est une étape de mélange physique et uniforme d'un mélange solidifié et d'un polymère biocompatible ayant une température de transition vitreuse de 80°C ou plus, dans lequel le mélange solidifié a été préparé en versant une solution, dans laquelle le matériau actif et un lipide en tant que lubrifiant sont dissous dans un solvant organique miscible à l'eau, dans de l'eau pour solidification ; filtration et séchage du mélange.

7. Le procédé de préparation de nanoparticules de matériau actif selon la revendication 1, dans lequel ladite étape (1) est une étape de mélange physique et uniforme d'un mélange solidifié et d'un polymère biocompatible ayant une température de transition vitreuse de 80°C ou plus avec de l'eau déminéralisée, dans lequel le mélange solidifié a été préparé en versant une solution, dans laquelle le matériau actif et un lipide en tant que lubrifiant sont dissous dans un solvant organique miscible à l'eau, dans de l'eau pour la solidification ; filtration et séchage du mélange.

8. Le procédé de préparation de nanoparticules de matériau actif selon la revendication 1, dans lequel ladite étape (1) est une étape de mélange physique et uniforme d'un mélange solidifié, d'un polymère biocompatible ayant une température de transition vitreuse de 80°C ou plus, et d'un ou plusieurs composants supplémentaires choisis parmi un polymère biocompatible ayant une température de transition vitreuse inférieure à 80°C, un tensioactif, et un agent anti-coagulation, dans lequel le mélange solidifié a été préparé en versant une solution, dans laquelle le matériau actif et un lipide en tant que lubrifiant sont dissous dans un solvant organique miscible à l'eau, dans de l'eau pour solidification ; filtration et séchage du mélange.

9. Le procédé de préparation de nanoparticules de matériau actif selon la revendication 1, dans lequel ladite étape (1) est une étape de mélange physique et uniforme d'un mélange solidifié, d'un polymère biocompatible ayant une température de transition vitreuse de 80°C ou plus, et d'un ou plusieurs composants supplémentaires choisis parmi un polymère biocompatible ayant une température de transition vitreuse inférieure à 80°C, un tensioactif, et un agent anti-coagulation ainsi que de l'eau déminéralisée, dans lequel le mélange solidifié a été préparé en versant une solution, dans laquelle le matériau actif et un lipide en tant que lubrifiant sont dissous dans un solvant organique miscible à l'eau, dans de l'eau pour solidification ; filtration et séchage du mélange.

10. Le procédé de préparation de nanoparticules de matériau actif selon la revendication 1, dans lequel ladite étape (1) est une étape de préparation d'un mélange solidifié en versant une solution, dans laquelle le matériau actif, un lipide en tant que lubrifiant et un polymère biocompatible ayant une température de transition vitreuse de 80°C ou plus sont dissous dans un solvant organique miscible à l'eau, dans de l'eau pour solidification ; et filtration et séchage du mélange.

11. Le procédé de préparation de nanoparticules de matériau actif selon la revendication 1, dans lequel ladite étape (1) est une étape de mélange physique et uniforme d'un mélange solidifié et d'un ou plusieurs composants supplémentaires choisis parmi un polymère biocompatible ayant une température de transition vitreuse inférieure à 80°C, un agent tensioactif, et un agent anti-coagulation, dans lequel le mélange solidifié a été préparé en versant une solution, dans laquelle le matériau actif, un lipide en tant que lubrifiant, et un polymère biocompatible ayant une température de transition vitreuse de 80°C ou plus sont dissous dans un solvant organique miscible à l'eau, dans de l'eau pour solidification ; filtration et séchage du mélange.

12. Le procédé de préparation de nanoparticules de matériau actif selon la revendication 1, dans lequel ladite étape (1) est une étape de mélange physique et uniforme d'un mélange solidifié et d'un ou plusieurs composants supplémentaires choisis parmi un polymère biocompatible ayant une température de transition vitreuse inférieure à 80°C, un agent tensioactif, et un agent anti-coagulation, ainsi que de l'eau déminéralisée, dans lequel le mélange solidifié a été préparé en versant une solution, dans laquelle le matériau actif, un lipide en tant que lubrifiant, et un polymère biocompatible ayant une température de transition vitreuse de 80°C ou plus sont dissous dans un solvant organique miscible à l'eau, dans de l'eau pour solidification ; filtration et séchage du mélange.

13. Le procédé de préparation de nanoparticule de matériau actif selon la revendication 1, dans lequel le processus de broyage dans ladite étape (2) est réalisé en continu en utilisant des rouleaux contre-rotatifs.

14. Le procédé de préparation de nanoparticules de matériau actif selon la revendication 1, dans lequel le retrait de lipide dans ladite étape (3) est réalisé en ajoutant en continu un fluide supercritique dans un réacteur contenant le produit résultant de ladite étape (2) et en le déchargeant de celui-ci.

15. Le procédé de préparation de nanoparticules de matériau actif selon la revendication 1, dans lequel le retrait des lipides en utilisant un fluide supercritique dans ladite étape (3) est réalisé sous une condition de pression de 50 atmosphères ou plus et une condition de température de 5 à 60°C.

16. Le procédé de préparation de nanoparticules de matériau actif selon l'une quelconque des revendications 1 à 15, dans lequel le matériau actif est choisi parmi les composants organiques, les composants organométalliques, les extraits naturels, les protéines, et leurs combinaisons.

17. Le procédé de préparation de nanoparticules de matériau actif selon l'une quelconque des revendications 1 à 15, dans lequel le lipide est choisi parmi les acides gras saturés ayant 10 à 22 atomes de carbone, les esters d'acide gras saturé ayant 10 à 22 atomes de carbone, les alcools gras saturés ayant 10 à 22 atomes de carbone, les mono-, di- ou tri-glycérides ayant un groupe acide gras saturé ayant 10 à 22 atomes de carbone, les hydrocarbures ayant 14 atomes de carbone ou plus, et leurs combinaisons.

18. Le procédé de préparation de nanoparticules de matériau actif selon l'une quelconque des revendications 1 à 15, dans lequel le polymère biocompatible ayant une température de transition vitreuse (Tg) de 80°C ou plus est un ou plusieurs choisis parmi un polymère biocompatible à base de cellulose ayant une Tg de 80°C ou plus, une polyvinylpyrrolidone ayant une Tg de 80°C ou plus, un alcool polyvinylique ayant une Tg de 80°C ou plus, et un polymère biocompatible à base d'Eudragit ayant une Tg de 80°C ou plus.
